# EUROPEAN PATENT SPECIFICATION

(11) **EP 2 552 912 B1**
(45) Date of publication and mention of the grant of the patent: **02.08.2017**
(21) Application number: 11713099.7
(22) Date of filing: 23.02.2011
(51) Int. Cl.: C07D 451/10

(54) **SYNTHESIS METHOD FOR PRODUCING TIOTROPIUM BROMIDE**
SYNTHESEVERFAHREN ZUR HERSTELLUNG VON TIOTROPIUMBROMID
PROCÉDÉ DE SYNTHÈSE POUR LA PRODUCTION DE BROMURE DE TIOTROPIUM

(30) Priority: 28.06.2010 TR 201005221; 01.04.2010 TR 201002520
(43) Date of publication of application: 06.02.2013
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2011/000051
(87) International publication number: WO 2011/123079

(56) References cited:
- EP-A1- 0 418 716
- WO-A1-03/057694
- WO-A1-2008/089852
- WO-A1-2009/087419
- HARRY H. WASSERMAN AND BRUCE H. LIPSHUTZ: "Reactions of lithium enolates with molecular oxygen alpha-hydroxylation of amides and other carboxylate derivatives", TETRAHEDRON LETTERS, vol. 16, 1975, pages 1731-1734, XP002614576, DOI: 10.1016/S0040-4039(00)72245-3

## Description

The present invention relates to a new method for producing (1α, 2β, 4β, 7β)-7-[(hydroxidi-2-thienylacetyl)oxy]-9,9-dimethyl-3-oxa-9- azoniatricyclo[3.3.1.0²⁴]nonane-bromide.

### Background of the Invention:

The compound which has the chemical name (1α, 2β, 4β, 7β)-7-[(hydroxidi-2-thienylacetyl)oxy]-9,9-dimethyl-3-oxa-9- azoniatricyclo[3.3.1.0^{2,4}]nonane-bromide is known as tiotropium bromide. This compound given in the formula 5 below was first disclosed in the patent numbered EP 418716.

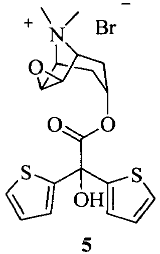

Tiotropium bromide is a highly effective anticholinergic agent. Due to this reason, it is commonly utilized in the treatment of asthma and COPD (chronic obstructive lung disease).

Tiotropium bromide is preferably used by the inhalation route. For the administration by the inhalation route, dry powder inhalers in which the dry powder is stored in capsules or blisters can be used. Another method comprises the administration of tiotropium bromide to patients in aerosol form with various gases (e.g. HFA134a and/or HFA227).

As tiotropium bromide is highly effective, even small amounts of it present therapeutic effects.

The patent numbered EP 418716 discloses a synthesis method for the preparation of tiotropium bromide demonstrated in Scheme 1.

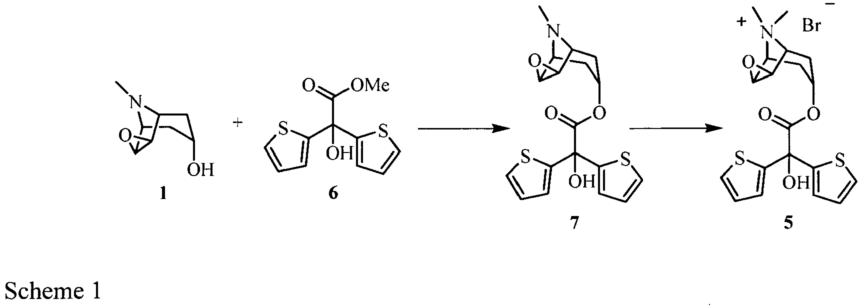

According to this method, scopine that is demonstrated in formula 1 reacts with di-(2-thienyl)-gylicolic acid methyl ester that is demonstrated in formula 6 and transforms into (2-thienyl)-gylicol acid scopine ester. Subsequently, tiotropium bromide is obtained by quaternizing the compound demonstrated in formula 7.

The first stage of this two-stage synthesis method is generally carried out at high temperatures like 70-90°C with hazardous chemicals such as sodium methoxide and metallic sodium. It is not preferable for manufacturers to carry out the reaction at high temperatures as it increases the cost. In addition, the efficacy of the reaction is in the range of 45-70% although it is realized under such tough conditions. All these indicate that synthesis methods with higher efficacy are needed in tiotropium bromide synthesis.

Also in this general area, WO 2009/087419 relates to processes for the preparation of scopine esters and quaternized scopine esters, WO 03/057694 is directed towards methods for producing quaternized scopine esters, and WO 2008/089852 relates to processes for preparing tiotropium salts.

The inventors have aimed to develop a high efficiency synthesis method which comprises few stages and eliminates the challenging purification stages, and enable to obtain high-purity tiotropium bromide.

### Detailed Description of the Invention

Surprisingly, it has been found that an easier and more effective obtain of tiotropium bromide is realized compared to the methods of tiotropium bromide in the prior art when the synthesis method pertaining to the present invention is applied. In one aspect, this synthesis method comprises the use of the procedure demonstrated in scheme 2.

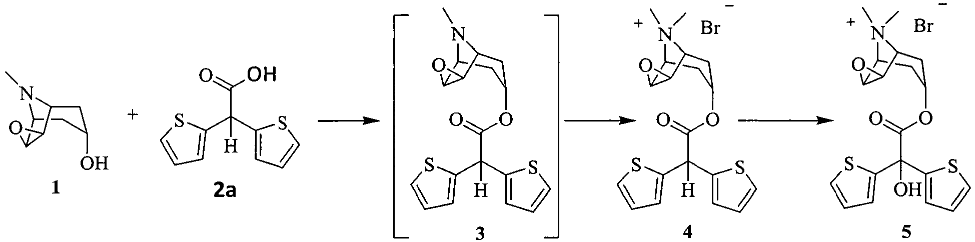

The inventors has surprisingly found that the final product in formula 4 is obtained with higher efficacy and purity when the compound demonstrated in formula 3 is transformed into formula 4 without being isolated.

Accordingly, the invention provides a method so as to be used in the synthesis of tiotropium bromide, wherein said method comprises the stages of;
i. obtaining the compound given in formula 3 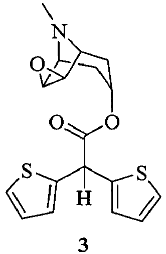 as a result of the reaction of scopine which is demonstrated in formula 1 or acid addition salts thereof 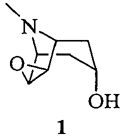 with the compound demonstrated in formula 2a, 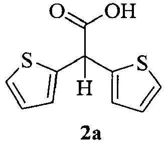
ii. transforming the substance, which is obtained after the solid substance in the reaction is removed through filtration, into formula 4 by quaternizing it 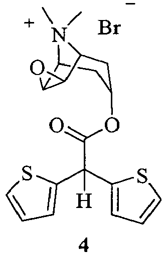
iii. transforming the quaternized scopine ester demonstrated in formula 4 into tiotropium bromide under oxidation conditions
and is characterized in transforming the compound that is illustrated in formula 3 into quaternized scopine ester (formula 4) without being subjected to any purification procedure;
wherein the method for the preparation of scopine ester (formula 3) comprises the stages of dissolving scopine (1) or an acid addition salt thereof and the compound illustrated in formula 2a in an organic solvent, and adding N,N'-dicyclohexylcarbodiimide (DCC), 1-Ethyl-3-(3-dimethylaminopropyl)carbodiimide (EDC) or N,N'-diisopropylcarbodiimide (DIC), and optionally 4-dimethylaminopyridine (DMAP) into the obtained solution.

According to this, one of the constituents of the present invention is the tiotropium bromide synthesis process in which the compound shown in formula 3 that is obtained as a result of the reaction of formula 1 and formula 2a is transformed into formula 4 under convenient conditions without being isolated.

The inventors have both decreased the cost of the manufacture by eliminating a purification process that is supposed to be used in the process and managed to obtain formula 4 with higher efficacy.

In one aspect, the present invention relates to a process so as to be used in the synthesis of tiotropium bromide and it comprises the steps of;
a) Obtaining the compound given in formula 3 as a result of the reaction of scopine which is demonstrated in formula 1 or acid addition salts thereof with the compound demonstrated in formula 2a,
b) Transforming the substance, which is obtained after the solid matter in the reaction mixture is removed through filtration, into formula 4 without being subjected to any purification processes,
c) Transforming the quaternized scopine ester demonstrated in formula 4 into tiotropium bromide under convenient oxidation conditions,
wherein the method for the preparation of scopine ester (formula 3) comprises the stages of dissolving scopine (1) or an acid addition salt thereof and the compound illustrated in formula 2a in an organic solvent, and adding N,N'-dicyclohexylcarbodiimide (DCC), 1-Ethyl-3-(3-dimethylaminopropyl)carbodiimide (EDC) or N,N'-diisopropylcarbodiimide (DIC), and optionally 4-dimethylaminopyridine (DMAP) into the obtained solution.

According to another aspect, the present invention relates to a method for preparation of tiotropium bromide and comprises the steps that scopine (1) or an acid addition salt thereof 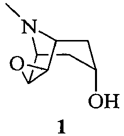 and the compound demonstrated in formula 2a, 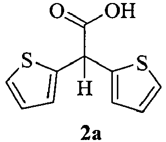 react; 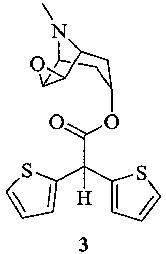 and scopine ester demonstrated in formula 3 is obtained at the end of the reaction; the compound illustrated in formula 4 is obtained by quaternizing the substance shown in formula 3 with methyl bromide at the second stage; formula 4 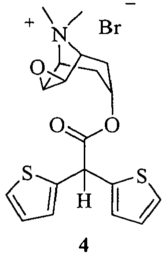 is subjected to oxidation reaction in oxygenic environment by using organic and/or inorganic basic substances and the final product tiotropium bromide is obtained at the third stage;
wherein the method for the preparation of scopine ester (formula 3) comprises the stages of dissolving scopine (1) or an acid addition salt thereof and the compound illustrated in formula 2a in an organic solvent, and adding N,N'-dicyclohexylcarbodiimide (DCC), 1-Ethyl-3-(3-dimethylaminopropyl)carbodiimide (EDC) or N,N'-diisopropylcarbodiimide (DIC), and optionally 4-dimethylaminopyridine (DMAP) into the obtained solution.

According to the present invention, scopine (1) can be in free form or in the form of an acid addition salt thereof, for instance hydrochloride, hydrobromide, hydrogenphosphate, hydrogensulphate, tetrafluoroborate and hexafluorophosphate salts. Preferably, it is used in free form.

According to this, the method for the preparation of scopine ester demonstrated in formula 3 requires scopine to dissolve in an appropriate organic solvent, for instance in dimethylformamide (DMF), dimetyl sulfoxide (DMSO), benzene, toluene, diethyl ether, tetrahydrofurane, ethanol, methanol, acetonitrile, acetone, ethyl acetate, methyl ethyl ketone, dichloromethane, dioxane, dimethylacetamide, N-methyl pyrrolidone, hexane, heptane or a combination thereof. Preferably, dimethylformamide or dichloromethane is used.

If scopine is used in the form of its acid addition salt, a base is added in order to reveal scopine. According to the present invention, said base can be selected from a group comprising organic and inorganic bases, for instance from the organic amines triethylamine, diisoprophylethylamine, pyridine, dimethylaminopyridine, N-methylprolidine, N-methyl morphorline or ammoniac. Preferably, ammoniac is used. The amount of said basic substance added is in the range of 1,25-2,5 moles per mole of scopine salt used, preferably in the range of 1,5-2,0 moles per mole of scopine salt used. The basic substance can be added at 0-60 °C, preferably at 15-50°C, most preferably at 20-30 °C. Following this, the mixture obtained is stirred at constant temperature for 0,5-3 hours, preferably for 1-2 hours. The salt which is produced during the reaction is filtered and removed. The solvent in the obtained solution is distilled under convenient temperature and pressure conditions. These conditions are determined based on the type of the solvent used.

Following this, the substance obtained is dissolved in an appropriate organic solvent, for instance in dimethylformamide (DMF), dimetyl sulfoxide (DMSO), benzene, toluene, diethyl ether, tetrahydrofurane, ethanol, methanol, acetonitrile, acetone, ethyl acetate, methyl ethyl ketone, dichloromethane, dioxane, dimethylacetamide, N-methyl pyrrolidone, hexane, heptane or a combination thereof, and then formula 2a is added.

The reaction may be realized in the presence of a base.

The base can be selected from a group comprising alkali carbonates or alkaline earth carbonates, lithium carbonate, sodium carbonate, potassium carbonate, calcium carbonate, sodium hydride, potassium hydride, calcium hydride, sodium methylate, sodium ethylate, potassium methylate and potassium ethylate. As the inorganic base, one of the hydrides is used. Most preferably, sodium hydride is used. The amount of said base used is at least at the stoichiometric proportion, preferably 1-3 moles, most preferably 1,5-2 moles per mole of scopine. The solution which is obtained after the base is added is stirred for 10-120 minutes, preferably for 30-90 minutes. The reaction may be realized at 40-90°C, preferably at 50-80°C, most preferably at 60-75°C and preferably under vacuum, and it is provided that the alcohol which is produced as a byproduct during the reaction is distilled and separated. Therefore, the balance of the reaction is readjusted towards scopine ester.

After the distillation is complete, the distilled solvent content of the reaction solution can be added again when necessary. The solution obtained is cooled to 5-40 °C, preferably to 0-35 °C, most preferably to 10-25 °C after the distillation is finished. The solution at this temperature is added hydrochloric acid for 12-120 minutes, preferably for 25-50 minutes. The hydrochloric acid used here can be in gas or aqueous solution form. However, it is preferred to be in aqueous solution form. Per one mole of scopine used, 80-350 ml of 36% hydrochloric acid, particularly 120-225 ml of 36% hydrochloric acid is added as dissolved in 10-20 liters of water, preferably in 12-17 liters of water.

Subsequent to the addition of hydrochloric acid solution, aqueous phase is separated. It is washed with a water immiscible organic solvent such as methylene chloride, ethyl acetate, toluene, n-butyl acetate, but preferably methylene chloride; organic phase is separated and removed. This step can be repeated if required.

The obtained aqueous phase is mixed with a water immiscible organic solvent such as methylene chloride, ethyl acetate, toluene, n-butyl acetate, and the pH value of the solution is adjusted in the range of 7,5-11, preferably in the range of 8-10 by adding an inorganic base into it, for instance carbonates of alkali metals or alkaline earth metals such as lithium carbonate, sodium carbonate, potassium carbonate, calcium carbonate, most preferably sodium carbonate. The inorganic base is preferably added in the form of aqueous phase.

According to this, 50-400 g, preferably 100-200 g of organic base per one mole is used as dissolved in 0,5-1,0 L, particularly preferably in 0,7-0,8 L of water.

After the obtained mixture is stirred, the organic phase is separated. The aqueous phase is extracted with a water immiscible organic solvent such as methylene chloride, ethyl acetate, toluene, n-butyl acetate. This step can be repeated if required. Then, the organic phases are combined and the organic solvent is distilled at an appropriate temperature and pressure to be removed.

The substance obtained upon distillation is dissolved in an appropriate solvent, for instance in a solvent combination which is comprised of dimethylformamide (DMF), dimethyl sulfoxide (DMSO), benzene, toluene, diethyl ether, tetrahydrofurane, ethanol, methanol, acetonitrile, acetone, ethyl acetate, methyl ethyl ketone, dichloromethane, dioxane, dimethylacetamide, N-methyl pyrrolidone, hexane, heptane or a combination thereof. The obtained solution is heated up to the boiling point of the solvent used, and then it is slowly cooled to -10-20 °C. Scopine ester which is obtained upon this procedure is filtered from the solution and dried under vacuum.

The present invention uses a compound of formula 2a, shown below:

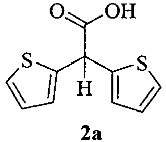

The synthesis method disclosed herein that will be used so as to obtain scopine ester illustrated in formula 3 comprises the steps of preparing scopine and acid addition salt thereof as described above; dissolving it in an appropriate solvent such as dimethylformamide (DMF), dimethyl sulfoxide (DMSO), benzene, toluene, diethyl ether, tetrahydrofurane, ethanol, methanol, acetonitrile, acetone, ethyl acetate, methyl ethyl ketone, dichloromethane, dioxane, dimethylacetamide, N-methyl pyrrolidone, hexane, heptane or a combination thereof; adding the compound of formula 2a into the obtained solution; and adding N,N'-dicyclohexylcarbodiimide (DCC), 1-Ethyl-3-(3-dimethylaminopropyl)carbodiimide (EDC) or N,N'-diisopropylcarbodiimide (DIC) and optionally 4-dimethylaminopyridine (DMAP) into the obtained solvent. Said reaction is stirred at -10-40 °C, preferably at room temperature for 10-48 hours, preferably for 12-16 hours.

In the said reaction, at least 1 mole, preferably 1,05-3 moles, most preferably 1,1-1,8 moles DCC, EDC or DIC is added per mole of scopine. Optionally, at least 0,01 mole, preferably 0,03-0,2 mole, most preferably 0,05-0,1 mole of DMAP per mole of scopine can be added.

In the case that solid particles remain in the solution at the end of the reaction, the solution is put through filtration. The solution that is obtained after the solid substance is filtered is diluted with an organic solvent, for instance dichloromethane, ethyl acetate, hexane, heptane, and it is extracted with water. After the organic phase is separated from the aqueous phase, the solvent used is removed under convenient conditions. The conditions to be used here, such as temperature and pressure, are chosen in accordance with the type of the solvent used.

Without being subjected to any purification operations, the obtained substance is dissolved in an appropriate organic solvent, for instance in a solvent combination which is comprised of dimethylformamide (DMF), dimethyl sulfoxide (DMSO), benzene, toluene, diethyl ether, tetrahydrofurane, ethanol, methanol, acetonitrile, acetone, ethyl acetate, methyl ethyl ketone, dichloromethane, dioxane, dimethylacetamide, N-methyl pyrrolidone, hexane, heptane or a combination thereof and the organic solution which contains 90-10%, preferably 30-60% of methyl bromide by weight is added into it. Then, it is stirred at 0-40 °C, preferably at room temperature for 12-90 hours, preferably for 18-72 hours and the quaternized scopine ester that is demonstrated in formula 4 is obtained.

According to another aspect, the present invention comprises the transformation of scopine ester which is demonstrated in formula 3 into quaternized scopine ester which is demonstrated in formula 4 without being subjected to any chromatographic purification processes.

The methyl bromide solution mentioned here is obtained through condensing methyl bromide gas at low temperature, then dissolving it in a desired organic solvent, for instance in a solvent combination which is comprised of dimethylformamide (DMF), dimethyl sulfoxide (DMSO), benzene, toluene, diethyl ether, tetrahydrofurane, ethanol, methanol, acetonitrile, acetone, ethyl acetate, methyl ethyl ketone, dichloromethane, dioxane, dimethylacetamide, N-methyl pyrrolidone, hexane, heptane or a combination thereof. Preferably, it is dissolved in acetonitrile solution.

If required, the obtained substance can be purified through one of the conventional purification methods such as anti-solvent crystallization, activated charcoal crystallization, thin layer chromatography (TLC), column chromatography, high pressure liquid chromatography (HPLC), distillation and the methods in the prior art.

The method for the preparation of tiotropium bromide which is illustrated in formula 5 is comprised of the stages of dissolving quaternized scopine ester demonstrated in formula 4 in an oxygen-saturated organic solvent, for example in a solvent that is comprised of dimethylformamide (DMF), dimethyl sulfoxide (DMSO), benzene, toluene, diethyl ether, tetrahydrofurane, ethanol, methanol, acetonitrile, acetone, ethyl acetate, methyl ethyl ketone, dichloromethane, dioxane, dimethylacetamide, N-methyl pyrrolidone, hexane, heptane or a combination thereof, preferably in acetonitrile or dichloromethane; adding an organic or an inorganic base such as potassium carbonate, sodium carbonate, potassium hydrogen carbonate, sodium tert-butoxide, triethyl amine into the obtained solvent; and stirring the obtained blend at -78-70 °C, preferably at -30-60 °C for 1-72 hours, preferably for 3-48 hours.

Another constituent of the present invention is that tiotropium bromide which is prepared according to the present invention is used in the production of a drug so as to be used in the treatment of many respiratory diseases, especially in asthma, chronic obstructive lung disease (COPD) and allergic rhinitis.

The synthesis method pertaining to the present invention can be explained with, but not limited to the following examples.

### Example 1: Process for the preparation of quaternized scopine ester (4)

Scopine and di-(2-thienyl) acetic acid are dissolved in dichloromethane. The obtained solution is added DCC and the mixture is stirred at room temperature for 16 hours. The solid substance in the reaction mixture is removed through filtration. The solvent in the obtained solution is removed under low pressure. The raw material obtained is dissolved in acetonitrile and added methyl bromide solution (50% MeBr, in acetonitrile). The tube containing the reaction mixture is closed and the obtained mixture is stirred at room temperature for 72 hours. The solid precipitate which comes out at the end is filtered, washed with a solvent, dried under low pressure and quartenized scopine ester **(4)** is obtained (80 % efficacy).

### Example 2: Process for the preparation of quaternized scopine ester (4)

Scopine and di-(2-thienyl) acetic acid are dissolved in dichloromethane and cooled to -10 °C. The solution prepared by dissolving DDC in dichloromethane is added to this mixture via a syringe, and then the reaction mixture is stirred at room temperature for 12 hours. The solid substance in the reaction mixture is removed through filtration and the solvent is removed under low pressure. The raw material obtained is dissolved in acetonitrile and added methyl bromide solution (50% MeBr, in acetonitrile). The tube containing the reaction mixture is closed and the obtained mixture is stirred at room temperature for 72 hours. The solid precipitate which comes out at the end is filtered, washed with a solvent, dried under low pressure and quartenized scopine ester **(4)** is obtained (76 % efficacy).

### Example 3: Process for the preparation of tiotropium bromide (5)

Quaternized scopine ester **(4)** and an organic base are dissolved in oxygene-saturated acetonitrile and the obtained mixture is stirred at room temperature for 48 hours. The solid precipitate which comes out at the end is filtered, washed with an appropriate solvent such as acetonitrile, dried under low pressure and tiotropium bromide **(5)** is obtained (74 % efficacy).

## Claims

1. A method so as to be used in the synthesis of tiotropium bromide, wherein said method comprises the stages of;
i. obtaining the compound given in formula 3 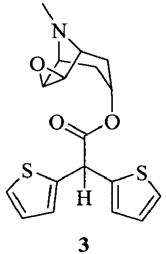 as a result of the reaction of scopine which is demonstrated in formula 1 or acid addition salts thereof 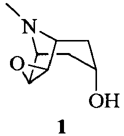 with the compound demonstrated in formula 2a 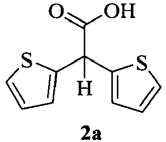
ii. transforming the substance, which is obtained after the solid substance in the reaction is removed through filtration, into formula 4 by quaternizing it 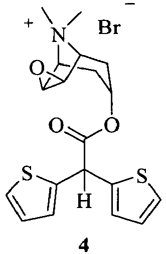
iii. transforming the quaternized scopine ester demonstrated in formula 4 into tiotropium bromide under oxidation conditions
and is **characterized in** transforming the compound that is illustrated in formula 3 into quaternized scopine ester (formula 4) without being subjected to any purification procedure;
wherein the method for the preparation of scopine ester (formula 3) comprises the stages of dissolving scopine (1) or an acid addition salt thereof and the compound illustrated in formula 2a in an organic solvent, and adding N,N'-dicyclohexylcarbodiimide (DCC), 1-Ethyl-3-(3-dimethylaminopropyl)carbodiimide (EDC) or N,N'-diisopropylcarbodiimide (DIC), and optionally 4-dimethylaminopyridine (DMAP) into the obtained solution.

2. The method for the preparation of scopine ester (formula 3) according to claim 1, wherein the organic solvent is chosen from a group comprising dimethylformamide (DMF), dimethyl sulfoxide (DMSO), benzene, toluene, diethyl ether, tetrahydrofurane, ethanol, methanol, acetonitrile, acetone, ethyl acetate, methyl ethyl ketone, dichloromethane, dioxane, dimethylacetamide, N-methyl pyrrolidone, hexane, heptane or a combination thereof.

3. The method for the preparation of scopine ester (formula 3) according to claim 1, wherein the amount of DCC, EDC or DIC added is at least 1 mole, per one mole of scopine used in said reaction.

4. The method for the preparation of scopine ester (formula 3) according to claim 1, wherein the amount of optionally added DMAP is at least 0,01 mole, per one mole of scopine in said reaction.

5. The synthesis method according to claim 1, wherein the method for the preparation of quaternized scopine ester (formula 4) comprises the steps of dissolving scopine ester (formula 3) and/or a acid addition salt thereof and the organic solvent containing 10-90%, preferably 30-60% of methylbromide in a solvent.

6. The synthesis method according to claim 5, wherein the method for the preparation of formula 4 comprises the use of scopine ester in free from or chosen from a group comprising acid addition salts thereof such as hydrochloride, hydrobromide, hydrogenphosphate, hydrogensulphate, tetrafluoroborate and hexafluorophosphate salts.

7. The synthesis method according to claim 1, wherein the method to be used for the preparation of tiotropium bromide (formula 5) 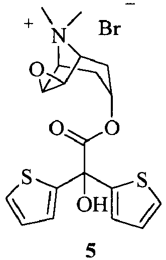 comprises the steps of dissolving quaternized scopine ester in an oxygen-saturated organic solvent and adding an organic or inorganic base into the solvent obtained.

## Patentansprüche

1. Verfahren für die Synthese der Tiotropiumbromid, umfassend die folgende Schritte;
i. das Erhalten der Verbindung in der Formel 3, 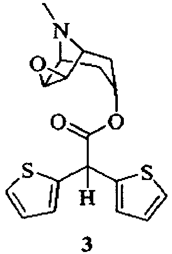 durch Reagiren der Skopin wie in Formel 1 oder in Säureadditionssalz davon gezeigt, 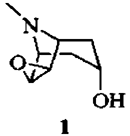 mit der in Formel 2a gezeigten Verbindung; 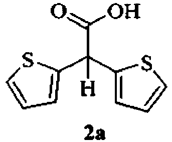
ii. die Umwandlung des Substanzes durch den quarternären Prozess in die Formel 4, nach dem der Feststoffes innerhalb der Reaktions durch Filtrieren gelöst wird, 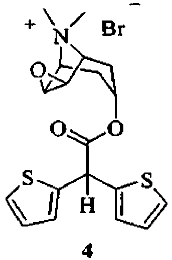
iii. die Umwandlung der unter Oxidationsbedingungen in Formel 4 gezeigten quarternären Skopin in Tiotropiumbromid
**dadurch gekennzeichnet, dass** die in Formel 3 gezeigte Verbindung in quarternären Scopinester (Formel 4) umgewandelt wird, ohne an einen zusätzlichen Reinigungsverfahren unterzogen wird,
wobei das Verfahren zur Herstellung von Scopinester (Formel 3) auch noch, das Lösen des Scopins (1) oder einer der Säureadditionssalze davon und der in Formel 2a bezeichnete Verbindung in einem organischen Lösungsmittel, das Hinzufügen der N, N'-Dicyclohexylcarbodiimid (DCC), 1-Ethyl-3- (3-dimethylaminopropyl) carbodiimid (EDC) oder N, N'-Diisopropylcarbodiimid (DIC) und optional 4-Dimethylaminopyridin (DMAP) in die erhaltene Lösung, umfasst.

2. Verfahren nach Anspruch 1 zur Herstellung der Scopinester (Formel 3), wobei das organische Lösungsmittel ausgewählt ist aus der Gruppe bestehend aus Dimethylformamid (DMF), Dimethylsulfoxid (DMSO), Benzol, Toluol, Diethylether, Tetrahydrofuran, Ethanol, Methanol, Acetonitril, Aceton, Ethylacetat, Methylethylketon, Dichlormethan, Dioxan, Dimethylacetamid, N-Methylpyrrolidon, Hexan, Heptan und einer deren Kombinationen.

3. Verfahren nach Anspruch 1 zur Herstellung der Scopinester (Formel 3), wobei die Menge der hinzugefügten der DCC, EDC oder DIC mindestens 1 Mol beträgt für ein Mol des in der erwähnten Reaktion verwendeten Scopins.

4. Verfahren nach Anspruch 1 zur Herstellung der Scopinester (Formel 3), wobei die Menge der optional hinzugefügten DMAP mindestens 0,01 Mol beträgt für ein Mol des Scopins.

5. Syntheseverfahren nach Anspruch 1, umfassend die Schritte das Lösen des Scopinesters des Verfahrens (Formel 3) zur Herstellung des quarternären Scopinesters (Formel 4) und/oder dessen Säureadditionssalzes und des Methylbromids im Bereich von 10-90 % Gew., vorzugsweise 30-60 % Gew in einem Lösungsmittel.

6. Syntheseverfahren nach Anspruch 5, umfassend das Anwenden des Verfahrens zur Herstellung der Formel 4 ausgewählt aus der Gruppe bestehend aus Säureadditionssalze wie Z.B. Hydrochlorid , Hydrobromid, Hydrogenphosphat, Hydrogensulfat , Tetrafluoroborat oder Hexafluorophosphat, oder Scopinester in freier Form.

7. Syntheseverfahren nach Anspruch 1, umfassend die Schritte; das Auflösen des quarternären Scopinesters im organsichen Lösungsmittel, das mit Oxygen gesättigte Verfahren zur Herstellung des Tiotropiumbromids (Formel 5) verwendet wird, 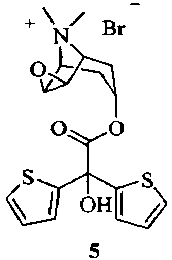 und das Hinzufügen der organischen oder inorganischen Base in die erhaltene Lösung.

## Revendications

1. Un procédé destiné à être utilisé dans la synthèse du bromure de tiotropium, dans lequel ledit procédé comprend les étapes de;
i. obtenir du composé donné dans la formule 3 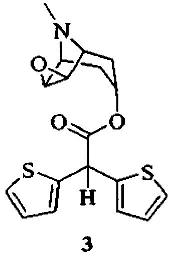 par suite de la réaction de la scopine qui est démontrée dans la formule 1 ou ses sels d'addition d'acide 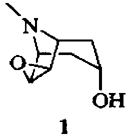 avec le composé démontré dans la formule 2a 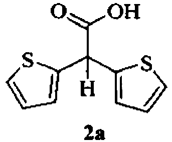
ii. transformer de la substance, qui est obtenue après élimination de la substance solide dans la réaction par filtration, dans la formule 4 en la quaternisant 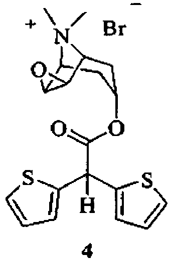
iii. transformer l'ester de scopine quaternisé démontré dans la formule 4 en bromure de tiotropium dans des conditions d'oxydation
et est caractérisé en transformant le composé illustré dans la formule 3 en ester scopine quaternisé (formule 4) sans être soumis à une procédure de purification;
dans lequel le procédé pour la préparation de l'ester scopine (formule 3) comprend les étapes de dissolution de scopine (1) ou d'un sel d'addition d'acide de celui-ci et le composé illustré dans la formule 2a dans un solvant organique et l'addition de N, N 'dicyclohexylcarbodiimide (DCC), 1-éthyl-3- (3-diméthylaminopropyl) carbodiimide (EDC) ou le N, N'-diisopropylcarbodiimide (DIC) et éventuellement la 4-diméthylaminopyridine (DMAP) dans la solution obtenue.

2. Procédé de préparation d'ester scopine (formule 3) selon la revendication 1, dans lequel le solvant organique est choisi parmi un groupe comprenant du diméthylformamide (DMF), du diméthylsulfoxyde (DMSO), du benzène, du toluène, de l'éther diéthylique, du tétrahydrofurane, de l'éthanol, du méthanol, de l'acétonitrile, de l'acétone, de l'acétate d'éthyle, de la méthyléthylcétone, du dichlorométhane, du dioxane, du diméthylacétamide, de la N-méthylpyrrolidone, de l'hexane, de l'heptane ou une combinaison de ceux-ci.

3. Procédé de préparation d'ester scopine (formule 3) selon la revendication 1, dans lequel la quantité de DCC, EDC ou DIC ajoutée est d'au moins 1 mole, par mole de scopine utilisée dans ladite réaction.

4. Procédé de préparation d'ester de scopine (formule 3) selon la revendication 1, dans lequel la quantité de DMAP éventuellement ajoutée est d'au moins 0,01 mole, par une mole de scopine dans ladite réaction.

5. Procédé de synthèse selon la revendication 1, dans lequel le procédé de préparation d'ester scopine quaternisé (formule 4) comprend les étapes consistant à dissoudre l'ester scopine (formule 3) et / ou un sel d'addition d'acide de celui-ci et le solvant organique contenant 10 à 90%, de préférence 30 à 60% de bromure de méthyle dans un solvant.

6. Procédé de synthèse selon la revendication 5, dans lequel le procédé de préparation de formule 4 comprend l'utilisation d'un ester scopine libre ou choisi parmi un groupe comprenant des sels d'addition d'acide tels que des sels de chlorhydrate, bromhydrate, hydrogénophosphate, hydrogénosulfate, tétrafluoroborate et hexafluorophosphate .

7. Procédé de synthèse selon la revendication 1, dans lequel le procédé à utiliser pour la préparation du bromure de tiotropium (formule 5) 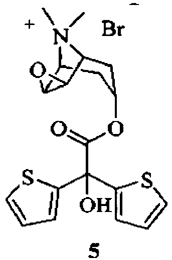 comprend les étapes consistant à dissoudre un ester scopine quaternisé dans un solvant organique saturé d'oxygène et à ajouter une base organique ou inorganique dans le solvant obtenu.
